# EUROPEAN PATENT APPLICATION

(11) **EP 1 479 369 A1**
(43) Date of publication of application: **24.11.2004**
(21) Application number: 03706961.4
(22) Date of filing: 19.02.2003
(51) Int. Cl.: A61K 7/06

(54) **TREATING AGENT FOR PROTECTING ANIMAL FIBER**

(30) Priority: 21.02.2002 JP 2002044462
(71) Applicant: Yugen Kaisha Okada Giken, Kakogawa-shi, Hyogo 675-0062 (JP)
(72) Inventor: OKADA, Toru, Kakogawa-shi, Hyogo 675-0062 (JP)
(74) Representative: Eisenführ, Speiser & Partner
(86) International application number: PCT/JP2003/001748
(87) International publication number: WO 2003/070206

(57) **Abstract**

A treatment agent for animal fiber protection which comprises an aqueous solution containing divalent metal ions or a divalent metal salt. The treatment agent prevents hair and animal fiber damage associated with permanent wave treatments, bleaching treatments, etc.

## Description

### TECHNICAL FIELD

The present invention relates to a treatment agent for animal fiber protection and an animal fiber treatment method that are designed to protect fibers from damage arising during the conduct of oxidation treatment for animal fibers consisting of keratin. The present invention relates to a hair treatment agent that is designed to protect hair especially in permanent wave treatment (perm treatment) and hair coloring treatment of human hair. The present invention also relates to a treatment agent that protects animal fibers from damage accompanying bleaching treatment of non-human animal fibers such as sheep's wool.

### BACKGROUND ART

Treatment using various chemicals is involved during so-called perm treatment in which human hair is permanently waved. With common perm treatments, cystine bonds in hair tissue are reduced with the aid of a first perming agent (thioglycolic acid) , as shown below.

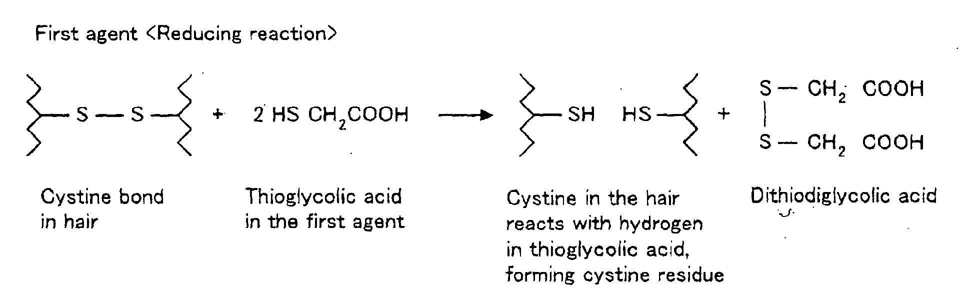

Next, after the hair has been made wavy, oxidation is conducted with the aid of a second agent (sodium bromate), and the cystine bond is reduced. After-treatment such as rinsing is then performed, and the perm treatment is completed.

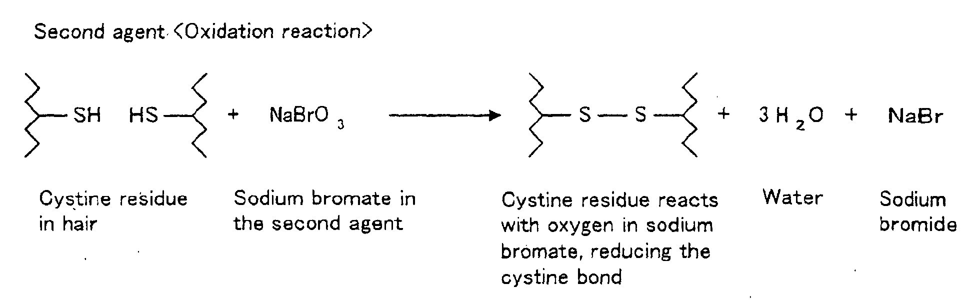

The above-mentioned second agent (sodium bromate) used in such perming treatment is an oxidizing agent. As shown below, cysteic acid is generated in that treatment process.

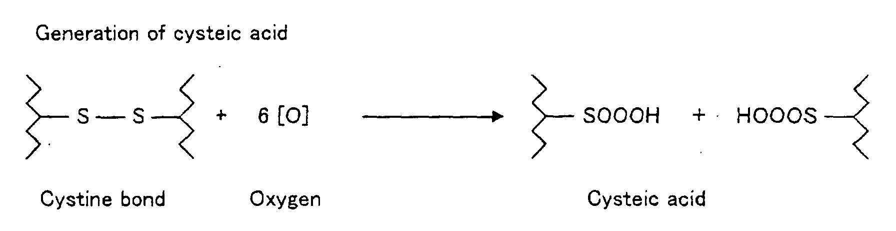

This cysteic acid is thought to reduce hair strength, and lead to hair damage or deterioration of feel of hair, or hair fading or decoloration. Effective methods to prevent hair quality reduction accompanying such perm treatments have not conventionally been known.

Cysteic acid is also similarly generated in head hair coloring treatment due to bleaching (oxidation treatment) using hydrogen peroxide associated therewith. Further, cysteic acid is also generated by oxidation treatment during bleaching treatment or hair coloring processes on animal fibers such as sheep's wool, cashmere, camel, and rabbit.

It is inferred that in the oxidation process associated with such perming treatment, etc, the cysteic acid generated largely impacts on the damage to hair. The present inventor earnestly examined methods to prevent damage of hair, or the like in such perming treatments, etc. As a result, the present invention was completed by acquiring the knowledge that by reacting divalent metals or their salts, such as calcium or calcium salts with cysteic acid and obtaining -SO₃Ca, the produced substance is inactivated, and insoluble and stable in water, and such damage can be prevented.

### DISCLOSURE OF THE INVENTION

Accordingly, the present invention provides a treatment agent for animal fiber protection comprising an aqueous solution containing divalent metal ions or a divalent metal salt. The present invention also provides a perming treatment method, hair coloring method, and bleaching method that are minimally damageable to animal fibers such as hair, using such protection treatment agent.

### BEST MODE FOR CARRYING OUT THE INVENTION

### (Divalent Metals)

It would be preferable for the metal component of the divalent metal ions and divalent metal salts used in the treatment agent for animal fiber protection in the present invention to be calcium, magnesium and barium. These metal ions and metal salts can be introduced in various forms into the treatment agent. For example, they can be combined as calcium, magnesium, and barium metal oxides (e.g. calcium oxide), hydroxides (e.g. calcium hydroxide), and chlorides (e.g. calcium chloride).

They can also be combined as calcium, magnesium, and barium organic acid salts (hydroxy acid metal salt, and fatty acid metal salt) , etc. Such metal hydroxy acid salts include aliphatic hydroxy acidmetal salts such as saccharic acids such as lactic acid, gluconic acid, ascorbic acid, citric acid, malic acid, glycolic acid, oxypropionic acid, tartaric acid, and glucaric acid, and aromatic hydroxy acid metal salts such as gallic acid, and hydroxybenzoic acid. Metal salts such as methyl vinyl ether-maleic anhydride and its copolymer calcium salt are also used.

They can also include polysaccharides and their derivatives, for example, metal salts such as the various calcium salts of carboxy methyl cellulose, hyaluronic acid, alginic acid, gellan gum, carboxy methyl chitin, and carboxy methyl chitosan.

### (Chelating agents)

Chelating agents may be added in order to assist dissolution of divalent metals. Such chelating agents could include ethylene diamine tetraacetate, nitrilo triacetate, diethylene triamine pentaacetate, hydroxy ethyl ethylene diamine triacetate, triethylene triamine hexaacetate, 1,3-propane diamine tetraacetate, 1,3-diamino-2-hydropropane tetraacetate, hydroxy ethylimino diacetate, dihydroxy ethyl glycine, glycol ether diamine tetraacetate, and dicarboxy methyl glutamic acid.

Of these, metal compounds such as calcium hydroxide, magnesium hydroxide, calcium lactate, magnesium lactate, calcium gluconate, calcium citrate, calcium malate, calcium saccharate, and calcium chloride in particular would be desirable, as would the combining thereof with a chelating agent such as ethylene diamine tetraacetate.

Based on metal conversion, the concentration of the above-mentioned metals or a metal salt in the treatment agent for animal fiber protection of the present invention is 80 to 50,000 mg/l, and preferably 100 to 20,000 mg/l, and even more preferably 200 to 8,000 mg/l. When the compound amount is less than this, the protective effect on animal fibers such as hair will be inadequate; when it is more than this, no improved effect will be seen, either.

Effective metals are preferably metals that are insoluble in water such as calcium in the form of -SO₃Ca. Magnesium compounds or the like are also preferable. Monovalentmetals such as potassium and sodium impede the effect of the present invention, so it would be preferable to use water with a low amount or no monovalent metal content such as pure water and ion exchange water.

### (Preparation of treatment agent for animal fiber protection)

The treatment agent for protection of the present invention can be prepared by sufficiently dissolving designated metal salts, etc in' an aqueous solution. It would also suffice to promote volatilization of the solvent by adding alcohol such as ethanol as desired. The treatment agent may be used in the form of a hair lotion as an aqueous solution, aqueous solution and hair mist, and could further be used in various forms including a hair spray using the aqueous solution in combination with a spray, foam, and gel.

Conventionally known ingredients such as plasticizers (e.g. polyethylene glycol), antioxidants, oils, preservatives, ultraviolet absorbers, thickeners, perfumes, and dyes may also be suitably combined as required in the treatment agent of the present invention provided they do not diminish its function.

Itmay also be used in the formof a shampoo, rinse, and treatment agents by formulation together with surface-active agents and conditioning ingredients.

The treatment agent for animal fiber protection of the present invention prevents damage and fading of animal fibers such as hair, and prevents hair quality reduction, by acting on cysteic acid that is generated in association with perm treatments, etc. It was able to markedly mitigate damage to animal fibers caused by cysteic acid.

### (Usage of Treatment Agent)

The usage of the treatment agent for animal fiber protection of the present invention is herein explained, using perm treatment for human hair as an example. In wave treatment, head hair is subj ect to treatment with a first agent, followed by wave treatment. This is followed by treatment with a second agent, which is then followed by rinsing. Next, as a stabilizing treatment, 10 to 200 ml, normally about 50 ml of an aqueous solution of dissolved divalent metal salt such as calcium salt is applied to the entire hair by an appropriate method such as a spray. After this solution has adhered to and permeated the hair for about 1 to 20 minutes, but preferably 2 to 10 minutes, it is removed by rinsing the hair with water.

### Examples

The present invention is herein further explained with reference to examples.

### [Example 1] (Hair Treatment Agent)

A hair treatment solution was prepared by adding 10 g of calcium hydroxide and 20 g of ethylene diamine tetraacetate to water, making up to 1 liter, and completely dissolving them therein.

### (Test Method)

A conventional wave perming treatment was performed on 10 g of cut hair. That is, first, reducing treatment was performed on the hair using the first agent (thioglycolic acid). Next, physical treatment such as heating was performed, and a wave perming process was conducted. Further, following oxidation treatment using the second agent (sodium bromate) , the hair was rinsed and the chemicals were removed. Twenty milliliters of the above-mentioned hair treatment agent was sprayed onto this hair and left for two minutes. Next, the treatment agent was removed by rinsing the hair with water and then the hair was dried. Defining each treatment as one process, the process was repeated once every week for a total of 10 times on the same hair. After 10 treatments a panel of five people evaluated macroscopically the extent of damage to the hair. The results are shown in Table 1. For comparison, a similar treatment was performed except insteadof the hair treatment agent, water (20 ml) was sprayed on hair (10 g) following perm treatment.

### [Examples 2 to 6] (Hair Treatment Agent)

Except for the use of the ingredients in Table 1, the hair treatment agent was prepared as in Example 1, and provided for evaluation. The results are shown in.Table 1.

**[ TABLE 1 ]**

| unit: g | | | | | | | |
|---|---|---|---|---|---|---|---|
| | Example | | | | | | Contrast |
| | 1 | 2 | 3 | 4 | 5 | 6 | |
| calcium lactate | - | 20 | - | - | - | - | - |
| magnesium lactate | - | - | 5 | - | - | - | - |
| calcium chloride | - | 0.1 | - | - | - | - | - |
| calcium gluconate | - | - | 5 | - | - | - | - |
| calcium saccharate | - | - | - | 10 | - | - | - |
| calcium citrate | - | - | - | 5 | - | - | - |
| calcium malate | - | - | - | - | - | 25 | - |
| calcium hydroxide | 10 | - | - | - | 5 | - | - |
| ethylenediamine tetraacetate | 20 | - | - | - | - | - | - |
| methyl vinyl ethermaleic anhydride copolymer | - | - | - | - | 20 | - | - |
| water | balance | balance | balance | balance | balance | balance | balance |
| evaluation | | | | | | | |
| panelist A | ⓞ | ⓞ | ⓞ | ⓞ | ⓞ | ⓞ | Δ |
| panelist B | ⓞ | ⓞ | ⓞ | ⓞ | ⓞ | ⓞ | Δ |
| panelist C | ⓞ | ⓞ | ⓞ | ⓞ | ⓞ | ⓞ | × |
| panelist D | ○ | ⓞ | ⓞ | ⓞ | ⓞ | ⓞ | Δ |
| panelist E | ⓞ | ⓞ | ⓞ | ⓞ | ⓞ | ⓞ | Δ |
| ⓞ: changeless ○: slight change Δ: somewhat change × : significant change | | | | | | | |

### [Examples 7 to 12] (Hair Treatment Agent)

A hair treatment solution was prepared by adding each of the constituent ingredients shown in Table 2 below to water, making up to 1 liter, and completely dissolving them therein. This hair treatment solution was evaluated similarly as described above. The results are also shown in Table 2.

**[ TABLE 2 ]**

| unit: g | | | | | | |
|---|---|---|---|---|---|---|
| | Example | | | | | |
| | 7 | 8 | 9 | 10 | 11 | 12 |
| gallic acid | 1 | - | - | - | 2 | - |
| lactic acid | - | - | - | - | - | 4 |
| triethylene triamine hexaacetate | 4 | - | - | - | - | - |
| hydroxy ethylimino diacetate | - | 5 | 3 | - | 5 | - |
| dihydroxy ethyl glycine | - | 0.5 | - | 4 | - | - |
| carboxy methyl cellulose | - | - | 0.5 | - | - | - |
| hyaluronic acid | - | - | 0.1 | - | - | - |
| methyl vinyl ether⁻ maleic anhydride copolymer | - | - | - | 4 | - | - |
| calcium hydroxide | 2 | 1 | 1 | 1 | - | - |
| magnesium hydroxide | - | - | - | - | 1 | 1 |
| water | balance | balance | balance | balance | balance | balance |
| evaluation | | | | | | |
| panelist A | ⓞ | ○ | ⓞ | ⓞ | ○ | ○ |
| panelist B | ⓞ | ⓞ | ⓞ | ⓞ | ○ | ○ |
| panelist C | ⓞ | ⓞ | ⓞ | ⓞ | ○ | ○ |
| panelist D | ⓞ | ⓞ | ⓞ | ⓞ | ○ | ○ |
| panelist E | ○ | ⓞ | ⓞ | ⓞ | ○ | ○ |

### [Examples 13 and 14, and Comparative Example 1] (Hair Coloring)

A hair treatment solution was prepared by adding each of the constituent ingredients shown in Table 3 below to water, making up to 1 liter, and completely dissolving them therein.

### (Test Method)

The process shown below comprising both a hair coloring treatment and perm treatment was repeated five times. Thereafter, the tensile strength of the hair was measured using a tensile tester. Strength was measured and the peak area was calculated based on the height of the peak of the chart obtained. The peak area represents the index of fiber toughness.

### (Process)

### (1) Shampoo treatment - (2) Rinsing with water - (3) Bleaching (room temperature) and Coloring treatment - (4) Rinsing with water - (5) Shampoo treatment - (6) Drying - (7) Wetting with water - (8) Perming with first agent (10 min at 32 C) - (9) Rinsing with water - (10) Perming with second agent (15 min at room temperature) - (11) Rinsing with water - (12) Drying

During the above processes, before and after (3) and in (12), the treatment agents for the examples and comparison example were used on the hair.

**[TABLE 3]**

| unit: g | | | |
|---|---|---|---|
| | Example | | Comparative ex |
| | 13 | 14 | 1 |
| gluconic acid | 5 | - | - |
| calcium oxide | 1 | 1 | - |
| dicarboxy methyl glutamic acid | - | 6 | - |
| water | balance | balance | whole |
| strength (g) | 110 | 115 | 80 |
| peak area (cm²) | 7.33 | 7.41 | 1.10 |

Hydrogen peroxide is used in the coloring treatment for hair. The use of this hydrogen peroxide generates cysteic acid, which leads to damage to the hair. However, the use of the protective treatment agent in the examples enabled the prevention of hair damage.

### [Examples 15 to 17, Comparative Example 2] (Coloring of Sheep's Wool)

A hair treatment solution was prepared by adding each of the constituent ingredients shown in Table 4 below to water, making up to 1 liter, and completely dissolving them therein. Degreasing using an alkaline (ammonia water) , and hydrogen peroxide treatment were conducted on individual 10 g cut wool pieces. Before and after these processes, treatment was performed using the above-mentioned protecting agent. The tensile strength of this wool was measured similarly as described above. The results are shown in Table 4.

**[TABLE 4]**

| unit: g | | | | |
|---|---|---|---|---|
| | Example | | | Comparative ex |
| | 15 | 16 | 17 | 2 |
| calcium gluconate | 4 | - | - | - |
| gluconic acid | 2 | - | - | - |
| glycol ether diamine tetraacetate | - | 5 | 5 | - |
| calcium hydroxide | - | 1 | - | - |
| magnesium hydroxide | - | - | 1 | - |
| water | balance | balance | balance | whole |
| strength (g) | 90 | 88 | 90 | 60 |
| peak area (cm²) | 3.51 | 3.55 | 2.91 | 0.90 |

As can be clearly seen from Table 4, the wool treated'with the treatment agent in the examples clearly shows greater fiber strength compared with that of the comparative example. Damage to animal fibers is therefore likely to be minimal when the treatment agent of the present invention has been used.

### INDUSTRIAL APPLICABILITY

The treatment agent for animal fiber protection of the present invention prevents hair and animal fiber damage associated with permanent wave treatments, bleaching treatments, etc.

## Claims

1. A treatment agent for animal fiber protection comprising an aqueous solution containing divalent metal ions or a divalent metal salt.

2. The treatment agent according to claim 1, wherein the divalent metal is calcium.

3. The treatment agent according to claim 1, wherein the divalent metal salt is a chelate-like substance selected from the group of calcium hydroxide, magnesium hydroxide, hydroxy acid salt, and ethylene diamine tetraacetate salt.

4. An animal fiber treatment method, wherein, in a process of conducting oxidation treatment for animal fibers consisting of keratin, the treatment agent according to claim 1 is brought into contact with the animal fibers before and/or after said treatment.

5. The treatment method according to claim 4, wherein, in a process of conducting permanent wave treatment for human hair, the treatment agent is brought into contact with the human hair before and/or after said wave treatment.

6. The treatment method according to claim 4, wherein, in a process of conducting coloring treatment for human hair, the treatment agent is brought into contact with the human hair before and/or after said coloring treatment.

7. The treatment method according to claim 4, wherein, in a process of conducting bleaching treatment of sheep's wool, the treatment agent is brought into contact with the sheep's wool before and/or after said bleaching treatment.
